(19) Europäisches Patentamt
European Patent Office
Office européen des brevets

(11) Publication number: **0 437 567 B1**

# (12) EUROPEAN PATENT SPECIFICATION

(45) Date of publication of patent specification: **07.12.94**

(51) Int. Cl.5: **C07C 229/36**, C07C 227/18, C07C 309/00, C07D 307/58

(21) Application number: **90910865.6**

(22) Date of filing: **25.07.90**

(86) International application number: **PCT/HU90/00052**

(87) International publication number: **WO 91/01969 (21.02.91 91/05)**

## (54) PHENYL-GLYCINE DERIVATIVES.

(30) Priority: **27.07.89 HU 522089**

(43) Date of publication of application: **24.07.91 Bulletin 91/30**

(45) Publication of the grant of the patent: **07.12.94 Bulletin 94/49**

(84) Designated Contracting States: **AT BE CH DE DK ES FR GB IT LI LU NL SE**

(56) References cited:
**EP-A- 0 009 609**
**US-A- 3 518 260**

**CHEMICAL ABSTRACTS, Vol. 95, no. 24, Dec 14, 1981, Columbus, Ohio, US; A. KAMADA et al.: "Study of enamine derivatives on phenylglycine as adjuvants for the rectal absorption of insulin", p. 374, col. 2, abstr. 209609m, Chem. Pharm. Bull. 1981, 29(7), 2012-19(Eng).**

(73) Proprietor: **CHINOIN Gyogyszer és Vegyészeti Termékek Gyára RT.**
**To utca 1-5**
**H-1045 Budapest IV (HU)**

(72) Inventor: **Bán, Károly**
**Ujvilág utca 37**
**H-1145 Budapest (HU)**
Inventor: **Bán, Annamária**
**Gogol utca 38**
**H-1133 Budapest (HU)**
Inventor: **Páli, Lajosné**
**Bán Tibor utca 5**
**H-1043 Budapest (HU)**
Inventor: **KRUPPA, Márta**
**Tamási t 32**
**H-1124 Budapest (HU)**
Inventor: **SOMFAI, Eva**
**Táncsics t 8**
**H-1014 Budapest (HU)**
Inventor: **HUSZAR, Csaba**
**Tamási t 32**
**H-1124 Budapest (HU)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

CHEMICAL ABSTRACTS, Vol. 94, no. 9, March 2, 1981, Columbus, Ohio, US; PALOMO COLL: "Enamines of amino acids and their silyl derivatives", p. 771, col. 2, abstr. 66063v, ES-A-480980

(74) Representative: Patentanwälte Schaad, Balass & Partner
Dufourstrasse 101
Postfach
CH-8034 Zürich (CH)

## Description

The present invention relates to new valuable intermediates suitable for the acylation of 6-amino-penicillanic acids and 7-cephalosporanic acid in the production of pharmaceutical compositions. The present invention further provides a process for the preparation of said intermediate compounds and an improved process to prepare the known Dane salts.

The substituents in the general formulae are as follows:

R stands for a $C_{1-2}$ alkyl group,

A stands for hydrogen, Me or $-SO_2CF_3-$, wherein Me stands for potassium or sodium ion

$R^1$ stands for $-CH_2-COOR$ or a group of the formula (IX)

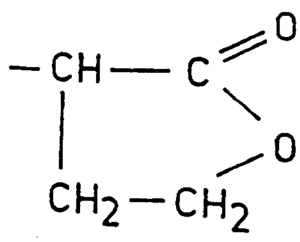

(IX)

$R^2$ stands for $=CH-COOR$ or a group of formula (XI)

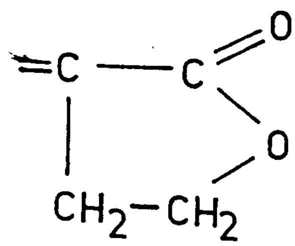

(XI)

The present invention relates to a process for preparing a compound of the general formula (I)

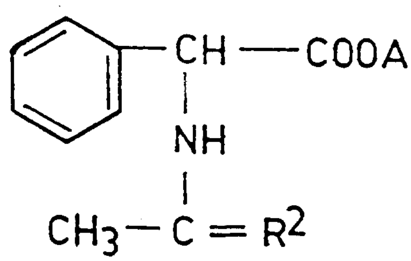

(I)

starting from an amino acid salt of the general formula (II)

(II)

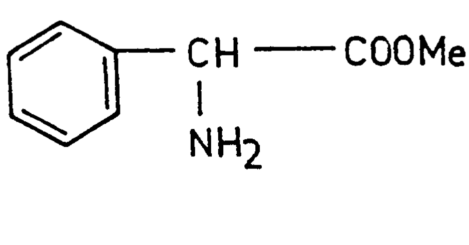

by forming a salt by reacting phenyl glycine of the formula (VII)

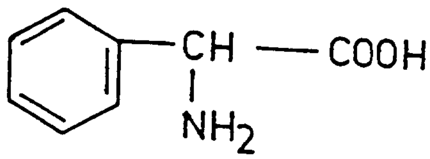

(VII)

with 1.05 - 1.3 mole of alkali-hydroxide in the presence of an alkanol and by reacting the reaction mixture with an acid having a pH value higher than 4.3 and by using 1.06 - 1.4 mole equivalent acid related to phenyl-glycine, the excess of alkali-hydroxide can be dissolved more selectively, while the amino acid salt of the general formula (II) precipitates from the solution or by reacting the reaction mixture with an equivalent amount of alkali salts of the acids having a pH-value above 4.3 and by condensing the amino acid salt of the general formula (II) obtained like described above or by any other method known per se in the presence of an alkanol with a ketone of the general formula (VIII)

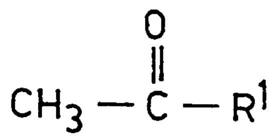

(VIII)

at the boiling point of the reaction mixture and removing continuously the formed water from the reaction mixture optionally by simultaneously adding a solvent by binary or ternary azeotrop distillation and upon cooling the reaction mixture the condensed salt of the general formula (IV)

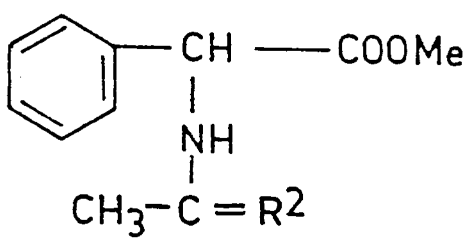

(IV)

can be isolated from the reaction mixture in crystalline form.

According to our invention a salt of the general formula (IV) can be reacted with a reactive derivative of the trifluoro-methane sulphonic acid of the formula (V)

$F_3C\text{-}SO_2OH$ (V)

preferably in the presence of a polar aprotic solvent.

The above processes can be used together or separately according to our present invention. The invention further extends to the novel compounds of the general formula, (VI)

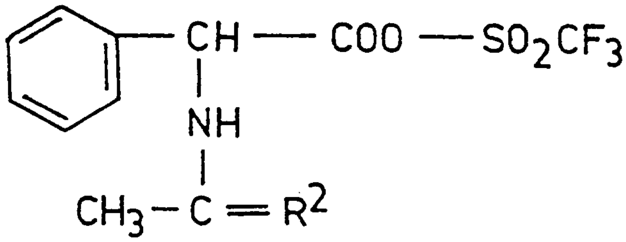

(VI)

These compounds can be prepared from intermediates prepared by known methods. The present invention provides also analogous processes in the course of which the known intermediates leading to new compounds were prepared by any known method.

The present invention further relates to analogous processes for the preparation of the novel compounds as well.

It is known that some of the beta-lactames acylated with D-2-phenyl-glycine are valuable pharmaceutically active compounds. Thus 6-amino-penicillanic acid acylated with D-2-phenyl-glycine is ampicilline and 7-amino-3-dezacetoxy-cephalosporanic acid acylated with the above amino acid is also known as a half-synthetic antibiotic called Cefalexin.

The above and similar synthetic antibiotics can be preferably prepared from D-2-phenyl-glycine by forming a potassium salt of N-(1-methyl-2-methoxy-carbonyl-vinyl)-D-2-phenyl-glycine or the potassium salt of N-(1-methyl-2-ethoxy-carbonyl-vinyl)-D-2-phenyl-glycine and preparing a mixed anhydride from the above compound and reacting same with a corresponding beta-lactame without isolation.

The enamine-salt prepared from D-2-phenyl-glycine was first prepared by Elizabeth Dane et al. [Chem. Ber. 98. 789-796 (1965)]. According to the process D-2-phenyl-glycine is boiled with the ethyl ester of aceto-acetic acid and with potassium-hydroxide and the product precipitating upon cooling is recrystallized from ethanol. Yield: 81.5 %. The obtained product contains half mole of crystal water which has to be removed before working up. According to Czechoslovakian patent application No. 147,194 the above reaction is carried out in ethanol. Yield: 75 %.

The products obtained according to the above processes can be worked up according to HU-PS 155,099 or DE-PS 3,012,669.

It is also known that the acylated derivatives of 6-amino-penicillanic acid and 7-amino-cephalosporanic acid can be used in the therapy as antibiotics of wide spectrum. One preferred representative of said compounds is D[(-)-$\alpha$-amino-(para-hydroxy-phenyl)-acetamido)]-penicillanic acid (referred to hereinafter as amoxicillin).

The criteria which amoxicillin has to meet are disclosed at pages 3-4. of the British Pharmacopoea 1973. One important criterium is the optical rotation of the product which is determined in the Pharmacopoea as: $(\alpha)_{20}^{D}$ = (+290°)-(+310°) (C=0.2 % by weight/volume, water). Amoxicillin can be prepared from 6-amino-penicillanic acid or salt thereof and from a derivative of para-hydroxy-phenyl-glycine according to GB-PS 978,178. According to GB-PS 1,339,605 6-amino-penicillanic acid is used in the form of its silylated derivative and the silyl group is removed after reaction. According to US-PS 3,674,776 the para-hydroxy-phenyl-glycine derivative containing a protected amino-group is reacted with 6-amino-penicillanic acid or salt thereof.

In the above patent specifications the mixed anhydrides of $\beta$-keto-esters formed by condensation of alkyl-esters of aceto-acetic acid are mentioned as preferred out of the reactive derivatives formed on the amino group. The preparation of said compounds can be performed by a reaction of para-hydroxy-phenyl-glycine with an alkali hydroxide, by separation of the formed alkali salts by reacting with an alkyl ester of aceto acetic acid and conversion of the enamine to a mixed anhydride, preferably with chloroformic acid esters. The disadvantage of the process is that the purity of Amoxicillin prepared from a mixed anhydride obtained like this does not achieve the requirements of the Pharmacopoea. The optical rotation of Amoxicillin prepared according to the Examples of US-PS 3,674,776 is only $(\alpha)_{D}^{20}$ = +246.5° (c=0.1 % in water). Purity of Amoxicillin in Example 1 of GB-PS 1,339,605 is 80 % by weight.

It is also known that Dane-salt was also prepared from para-hydroxy-phenyl-glycine in methanol at the boiling point of the reaction mixture using an ester of aceto acetic acid and replacing the mixture by toluene at the end of the reaction and by crystallizing the product from toluene. This method using toluene can not be reproduced in industry because by increasing the size the mixture contains more and more unreacted alkali salt of amino acid and cannot be filtered.

In order to eliminate partially the above problems it has been suggested to give a lye to the system when preparing Dane-salt (HU-PS 182,519) or to use isopropanol as an alkanol (HU-PS 186,143) by maintaining a narrow temperature range (65 - 70 °C).

At a temperature higher than this a reesterification takes place. The above processes did not result the hoped improvement on industrial scale.

In each further step the mixed anhydride was formed with a chlorocarbonic acid ester forming an acylating agent.

The mixed anhydrides formed from Dane-salt and chlorocarbonic ester can be well used for peptide chemical synthesis, but the preparation thereof is not without problem. Thus they can be prepared only at very low temperature (about -20 °C) and when using the very toxic chlorocarbonic esters in industrial circumstances a great care has to be paid and the costs are high.

The novel triflates of the general formula (VI) of the present invention are compounds which can be prepared relatively easily and are well determined and useful for the elimination for the above problems.

As a first step of the process of the present invention phenyl glycine of the formula (VII) can be reacted preferably with potassium or sodium hydroxide in the presence of methanol or ethanol.

The reaction mixture is reacted preferably with acetic acid or propionic acid separating thereby the excess of alkali hydroxide.

According to our best knowledge we have as first in the chemical literature prepared the phenyl glycine salt without residual lye and thereby both the salt and later the Dane-salts can be stabilized. The basis of this process is that we have recognized that by adding an acid with appropriate pK-value the contaminating lye excess can be selectively separated from the amine salt precipitating in crystalline form (the addition of which can not be avoided if the amine salt has to be prepared with a suitable yield).

In the course of the process of the invention about 10 percent more acid is used than would be necessary in order to bind the excess lye. This is due to the recognition that the presence of a catalytical amount of acid catalyzes the condensation reaction.

According to our invention we have first prepared such Dane-salt which was not prepared with an ester of aceto acetic acid but by using butyro lactone. This product can be similarly applied like the classical Dane-salt prepared with the ester of aceto-acetic acid. The preparation and utilization is justified if the butyro lactone is of some reason easier accessable or cheaper than the aceto acetate.

The $\beta$-keto-compound of the general formula (VIII) is preferably used in an excess of about 30 % by weight.

A further subject of the present invention is the removal of the water formed during the reaction when preparing Dane-salt by both methods.

We have recognized that Dane-salts crystallize with half a mole of water. This has been not mentioned in the literature. Therefore the later mixed anhydride formation can be performed with more difficulties.

We have found that water obtained in the condensation reaction also appears in the form of crystal water. Several methods are given for removing water depending on the solvent used for the reaction sequence considering economical and other points of view.

We can proceed by performing the reaction in the presence of methanol and distilling off the methanol at the end of the reaction by adding to the reaction mixture a solvent which forms with water a binary mixture of minimal boiling point and removing the water by azeotrop distillation. It is preferred to add lower carboxylic acid esters or aromatic hydrocarbons, preferably diethyl carbonate, alkyl acetates, benzene, toluene, xylene or acetonitrile, and removing the water in the form of a binary mixture formed with said solvents.

If the reaction is performed in the presence of ethanol then the water formed during the reaction can be removed from the reaction mixture by azeotrop distillation. It can be more economical, if the ethanol is expensive to perform the condensation reaction in the presence of ethanol and adding at the end of the reaction a solvent which forms with water and ethanol a ternary mixture of minimal boiling point. Then the water is removed by ternary azeotrop distillation. As such solvents aromatic hydrocarbons or esters etc. can be mentioned.

In order to prepare triflate one can preferably proceed by suspending the filtered cake-wet or dried Dane-salt in acetonitrile or nitro-methane and reacting same at a temperature of 0 °C with trifluoro-methane-sulphonic acid-chloride added in small portions and without using any acid binding agent (see DE-PS 2,613,172 where the reaction is performed at -20 - (-30) °C in the presence of N-methyl-morpholine). The obtained mixed anhydride is well soluble in the applied solvent and can be separated by filtration from the alkali chloride formed in the reaction. The obtained solution can be directly used for further reaction and the active ingredient content amounts to 90 % by weight according to HPLC analysis.

The acetonitrile solution can be diluted with ether or dichloro-methane and the anhydride can be isolated in solid form.

In order to prepare enamines of homogeneous composition and in order to avoid reesterification it is desireable to use such alcohol which esterifies the aceto-acetic acid.

The invention is illustrated by the following, non-limiting examples.

**Example 1**

18 g of sodium hydroxide are dissolved in 600 ml of ethanol and 67 g of D(-)-phenyl-glycine are added. After stirring for 15 minutes at 60 °C a clear solution is obtained. Now 2 ml of acetic acid and 75 g of acetic acid ethylester are added and the mixture is refluxed for 1.5 hours and 330 ml fraction is distilled off. The suspension obtained is cooled to -5 °C, the crystals are filtered and dried at 60 °C. 126.8 g of sodium-D-N-(2-ethoxy-carbonyl-1-methyl-vinyl)-α-amino-phenyl-acetate are obtained. Active ingredient: 98.2 %.
$[\alpha]_D^{20}$ = -84.5° (c = 1, n HCl)
Quality (stored at room temperature) is unchanged after 30 days.

**Example 2**

To 600 ml of ethanol 40 g of sodium acetate and 67 g of D(-)-phenyl-glycine are added. The reaction mixture is boiled for 2 hours whereafter 75 g of acetacetic acid ethylester are added and the mixture is refluxed for 1.5 hours. A 33 ml fraction is distilled off and the remainder is cooled to -5 °C. After filtration the crystals obtained are dried in vacuo at 60 °C. 124.5 g of sodium-D-N-(2-ethoxy-carbonyl-1-methyl-vinyl)-α-amino-phenyl-acetate are obtained.
Active ingredient: 98.2 %
$[\alpha]_D^{20}$ = -84.5° (c = 2, n HCl)

**Example 3**

In 600 ml of methyl alcohol 28.5 g of potassium hydroxide are dissolved, whereafter to the solution obtained 75 g of D(-)α-phenylglycine are added. The mixture is stirred for 15 minutes at 60 °C until a solution is obtained. The solution is mixed with 75 g of aceto-acetic acid methylester and it is refluxed for 2 hours. Thereafter the pH-value is adjusted to neutrality by adding acetic acid and at atmospheric pressure 400 ml of methyl alcohol are distilled off. The distillation is carried out in a way that the distilled fraction is continuously replaced by an identic volume of toluene until the inner temperature is reaching 110 °C. Thereafter the suspension obtained is cooled to 20 °C, the crystals are filtered and washed with toluene and dried in vacuo at 60 °C. 130 - 132 g of potassium-N-(1-methoxycarbonyl-1-methyl-vinyl)-α-amino-phenyl-acetate are obtained. (D-phenylglycine-Dane-salt).

| Analysis: | C | H | N |
|---|---|---|---|
| calculated: | 54.35 | 4.88 | 4.88 |
| found: | 54.31 | 4.86 | 4.90. |

The pH-value of a 5 % aqueous solution is neutral.

b.) 28.7 g of the D (-)-phenyl-glycine-Dane-salt are suspended in 150 ml of acetonitrile and at 0 °C 17 g of trifluoro-methane sulphonic acid chloride are dropwise added. After addition the reaction mixture is stirred at 0 °C for 30 minutes and thereafter it is allowed to reach a temperature of 20 °C. Then it is filtered and the acetonitrile solution obtained (active ingredient content: 95 % HPLC) is used in the peptide reaction.

**Example 4**

**a.)** In 600 ml of methyl alcohol 28.5 g of potassium-hydroxide are dissolved, thereafter 75 g of D (-)-α-phenylglycine are added. After stirring at 60 °C for 15 minutes 85 g of α-acetyl-γ-butyrolactone are added and the mixture is boiled for 90 minutes and the pH-value is adjusted by acetic acid to neutrality. 200 ml of methyl alcohol are distilled off and the remainder is cooled to 0 °C. The suspension obtained is filtered, the crystals are washed with cold methyl alcohol and dried in vacuo.

134 g of potassium-N-[2-(2-oxo-4,5-dihydrofuran-3-en)-ethyl]-α-amino-phenyl-acetate are obtained.

| Analysis: | C | H | N |
|---|---|---|---|
| calculated: | 56.19 | 4.68 | 4.68 |
| found: | 56.01 | 4.71 | 4.66 |

b.) From the salt obtained above 30 g are suspended in 150 ml of acetonitrile and at 0 °C 17 g of trifluoro-methane sulphonic acid chloride are dropwise added. The reaction mixture is stirred at this temperature for 30 minutes and thereafter the temperature is allowed to reach 20 °C. After filtration the acetonitrile solution containing a mixed anhydride (active ingredient: 90 %) is applied immediately for the peptide reaction.

## Claims

1. A process for the preparation of compounds of the general formula (I)

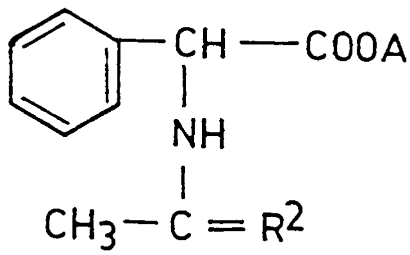

(I)

wherein

A is a hydrogen atom, Me or a $SO_2CF_3$ group, where Me is a potassium or sodium ion -
$R^2$ is =CH - COOR or a group of the formula (XI),

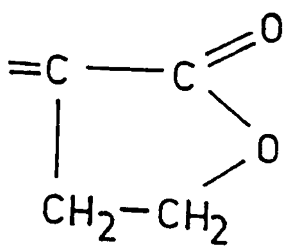

(XI)

R is a $C_{1-2}$ alkyl group -
from an amino acid salt of the general formula (II)

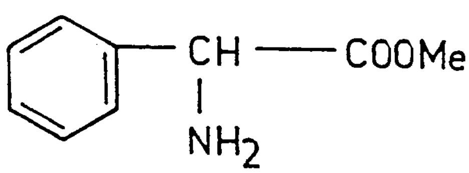

(II)

Me is as given above -
which comprises reacting the phenyl-glycine of the formula (VII)

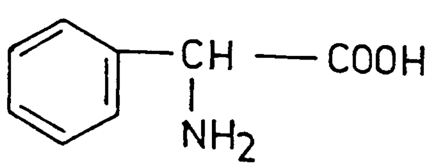

(VII)

in the presence of alkanol with 1.05 - 1.3 moles of alkali hydroxide to form a salt and reacting the mixture with an acid with a higher pH value than 4.3 and applying 1.06 - 1.4 moles of equivalent acid calculated on phenyl-glycine, dissolving the alkali hydroxide excess selectively, while the amino acid salt of the general formula (II) - Me is as mentioned above - is precipitated from the solution, or carrying out the reaction with an equivalent amount of alkali salts of acids possessing a higher pH value than 4.3, and condensing the amino acid salt of the general formula (II) obtained this way or by a process known per se

- Me is as mentioned above -
  in the presence of alkanol with a compound of the general formula (VIII)

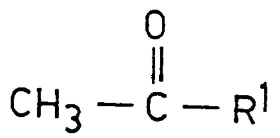

(VIII)

- $R^1$ is $-CH_2-COOR$ or a group of the formula (IX)

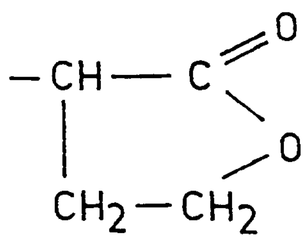

(IX)

- R has the same meaning as mentioned above -
  at the boiling point of the reaction mixture, while the water formed in the reaction - occasionally by addition of a solvent - is removed continuously from the reaction mixture by a binary or ternary azeotropic distillation and isolating the condensed salt of the general formula (IV)

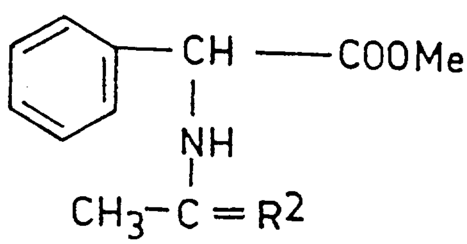

(IV)

- $R^2$ and Me are as mentioned above - in a crystalline form from the reaction mixture by cooling the mixture and drying and/or reacting the salt of the general formula (IV) obtained this way or by an other process
- $R^2$ and Me are as mentioned above - with a reactive trifluoro-methane sulphonic acid derivative of the formula (V),

  $F_3C-SO_2OH$ (V)

  preferably in the presence of a polar aprotic solvent.

**2.** Process according to claim 1, **characterized** by that the alkali hydroxide excess is separated by reacting the reaction mixture with acetic or propionic acid.

**3.** Process according to any of claims 1 or 2, **characterized** by, that the condensation is carried out in the presence of methyl alcohol and when completing the reaction the methyl alcohol is distilled off by giving to the reaction mixture a solvent, which with water forms a binary mixture of the lowest boiling point and the water is removed by azeotropic distillation.

**4.** Process according to claim 3, **characterized** by, that lower esters or aromatic hydrocarbons, advantageously diethylcarbonate, alkyl acetates, benzene, toluene, xylene or acetonitrile are added to remove the water in the form of a binary mixture.

**5.** Process according to any of claims 1 or 2, **characterized** by, that the condensation is carried out in the presence of ethyl alcohol and from the reaction mixture the formed water is removed by a binary azeotropic distillation.

**6.** Process according to any of claims 1 or 2, **characterized** by, that the condensation is carried out in the presence of ethyl alcohol and at the end of the reaction such a solvent is added, which forms with water and ethyl alcohol a mixture of a minimal boiling point and the water is removed by azeotropic distillation.

**7.** A process according to claim 6, **characterized** by, that in addition of ethyl alcohol as solvents ethyl-acetate, benzene or butyl acetate are used.

**8.** Process for the preparation of compounds of the general formula (VI)

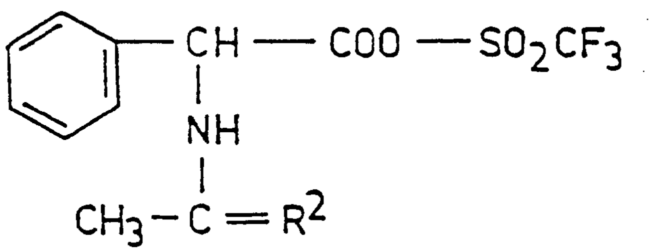

(VI),

$R^2$ is =CH-COOR or a group of the formula (XI)

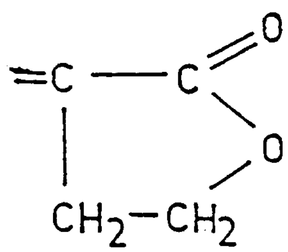

(XI),

wherein

R means a $C_{1-2}$ alkyl group -

**characterized** by, that a condensed salt of the general formula (IV)

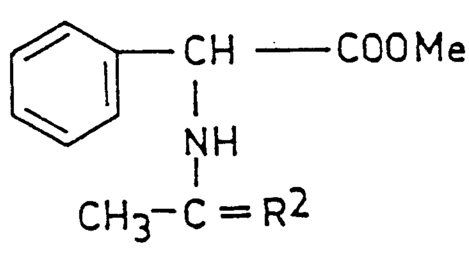

(IV),

wherein $R^2$ has the meaning mentioned above and Me means Na or K is reacted with a reactive trifluoro-methane sulphonic acid derivative, preferably with its chloride.

**9.** Process according to claim 8, **characterized** by, that the condensed salt is reacted at a temperature of 0 - 5 °C with trifluoro-methane sulphonic acid chloride.

**10.** New compounds of the general formula (VI)

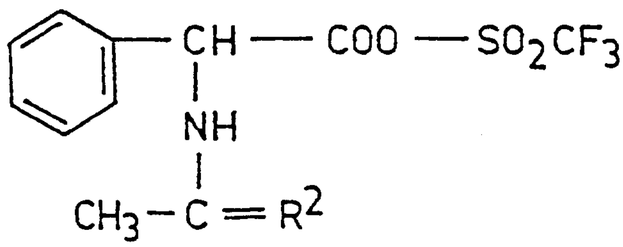

(VI),

wherein

$R^2$ is =CH-COOR or a group of the formula (XI)

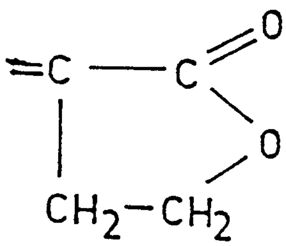

(XI),

and R is a $C_{1-2}$ alkyl group.

**Patentansprüche**

**1.** Verfahren zur Herstellung von Verbindungen der allgemeinen Formel (I) :

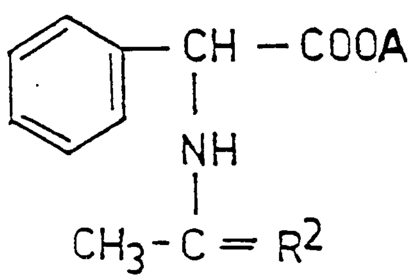

(I)

worin

A Wasserstoff, Me oder $-SO_2CF_3$ bedeutet, - wobei Me Kalium oder Natrium ist -

$R^2$ eine =CH-COOR-Gruppe - in der R ein $C_{1-2}$-Alkyl ist - oder eine Gruppe der Formel (XI):

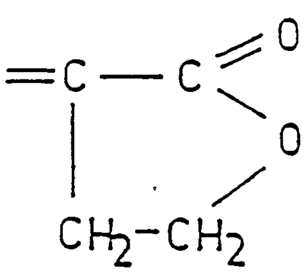

(XI)

bedeutet,
aus einem Aminosäuresalz der allgemeinen Formel (II):

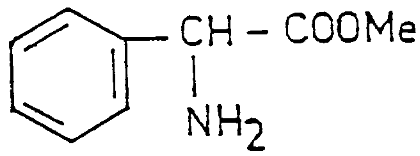

(II)

worin Me die oben angegebene Bedeutung hat,
dadurch gekennzeichnet, daß man das Phenylglycin der Formel (VII):

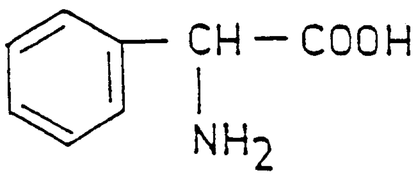

(VII)

in Gegenwart von Alkanol durch Umsetzen mit 1,05-1,3 Mol Alkalihydroxid in ein Salz überführt, und die so erhaltene Mischung mit 1,06-1,4 Moläquivalenten - auf das Phenylglycin bezogen - einer Säure, die einen pH-Wert von mehr als 4,3 hat, behandelt, das überschüssige Alkalihydroxid selektiv in Lösung geht, während das Aminosäuresalz der allgemeinen Formel II - worin Me die oben angegebene Bedeutung hat - aus der Lösung ausfällt, oder
die Reaktion mit einer äquivalenten Menge von Alkalisalzen von Säuren, die einen pH-Wert von mehr als 4,3 haben, durchführt und das auf diese Weise oder nach einem an sich bekannten Verfahren erhaltene Aminosäuresalz der allgemeinen Formel II - worin Me die oben angegebene Bedeutung hat - in Gegenwart von Alkanol mit einer Verbindung der allgemeinen Formel (VIII):

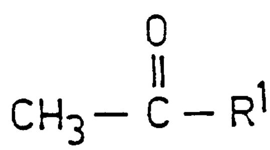

(VIII)

worin $R^1$ eine $-CH_2-COOR$-Gruppe - in der R die oben angegebene Bedeutung hat - oder eine Gruppe der Formel (IX):

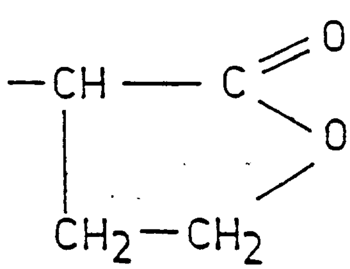

(IX)

bedeutet,
beim Siedepunkt der Reaktionsmischung kondensiert, während das in der Reaktion gebildete Wasser gelegentlich durch Zusatz eines Lösungsmittels kontinuierlich aus der Reaktionsmischung mittels einer binären oder ternären azeotropen Destillation entfernt wird, und das durch die Kondensation erhaltene Salz der allgemeinen Formel (IV):

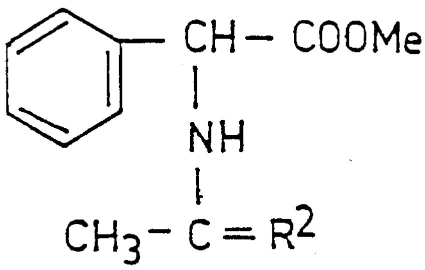

(IV)

worin $R^2$ und Me die oben angegebene Bedeutung haben, in kristalliner Form aus der Reaktionsmischung durch Kühlen der Mischung und Trocknen isoliert, und / oder das so oder nach einem anderen Verfahren erhaltene Salz der allgemeinen Formel IV, - in der $R^2$ und Me die oben angegebene Bedeutung haben - mit einem reaktiven Trifluormethan-sulfonsäurederivat der Formel (V):

$F_3C\text{-}SO_2OH$ (V)

vorzugsweise in Gegenwart eines polaren aprotischen Lösungsmittels, umsetzt.

2. Verfahren nach Anspruch 1, dadurch gekennzeichnet, daß man das überschüssige Alkalihydroxid durch Behandlung der Reaktionsmischung Essig- oder Propionsäure, abtrennt.

3. Verfahren nach einem der Ansprüche 1 oder 2, dadurch gekennzeichnet, daß man die Kondensation in Gegenwart von Methylalkohol durchführt, den man zur Vervollständigung der Reaktion abdestilliert, indem man der Reaktionsmischung ein Lösungsmittel zusetzt, das mit Wasser eine binäre Mischung mit einem Minimumsiedepunkt bildet, und das Wasser durch azeotrope Destillation entfernt.

4. Verfahren nach Anspruch 3, dadurch gekennzeichnet, daß man niedere Ester oder aromatische Kohlenwasserstoffe, vorzugsweise Diethylcarbonat, Alkyl-acetat, Benzol, Toluol, Xylol oder Acetonitril, der Reaktionsmischung zusetzt, und das Wasser in Form einer binären Mischung entfernt.

5. Verfahren nach Anspruch 1 oder 2, dadurch gekennzeichnet, daß man die Kondensation in Gegenwart von Ethylalkohol durchführt und das gebildete Wasser aus der Reaktionsmischung mittels einer binären azeotropen Destillation entfernt.

6. Verfahren nach Anspruch 1 oder 2, dadurch gekennzeichnet, daß man die Kondensation in Gegenwart von Ethylalkohol durchführt und am Ende der Reaktion ein Lösungsmittel zusetzt, das mit Wasser und Ethylalkohol eine Mischung mit einem Minimumsiedepunkt bildet, und das Wasser durch azeotrope Destillation entfernt.

7. Verfahren nach Anspruch 6, dadurch gekennzeichnet, daß man zusätzlich zum Ethylalkohol als Lösungsmittel Ethylacetat, Benzol oder Butylacetat verwendet.

8. Verfahren zur Herstellung von Verbindungen der allgemeinen Formel (VI):

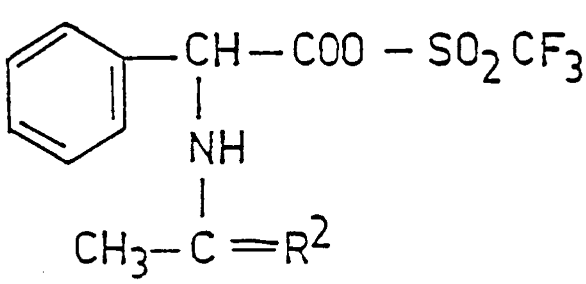

(VI)

worin

$R^2$ eine =CH-COOR-Gruppe - in der R ein $C_{1-2}$-Alkyl ist - oder eine Gruppe der Formel (XI):

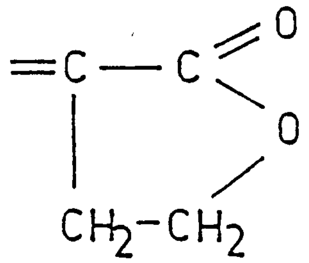

(XI)

bedeutet,

dadurch gekennzeichnet, daß man das kondensierte Salz der allgemeinen Formel (IV):

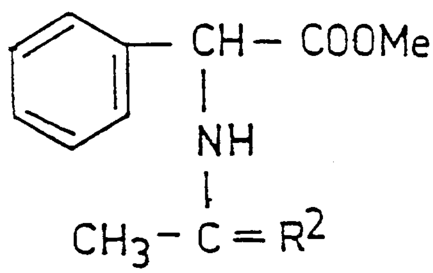

(IV)

worin

$R^2$ die oben angegebene Bedeutung hat und

Me Natrium oder Kalium ist,

mit einem reaktiven Trifluormethan-sulfonsäurederivat der Formel (V):

$F_3C\text{-}SO_2OH$ (V)

vorzugsweise seinem Chlorid, umsetzt.

**9.** Verfahren nach Anspruch 8, dadurch gekennzeichnet, daß man die Reaktion zwischen dem kondensierten Salz und dem Trifluotmethansulfonsäure-chlorid bei einer Temperatur von 0-5 °C durchführt.

**10.** Neue Verbindungen der allgemeinen Formel:

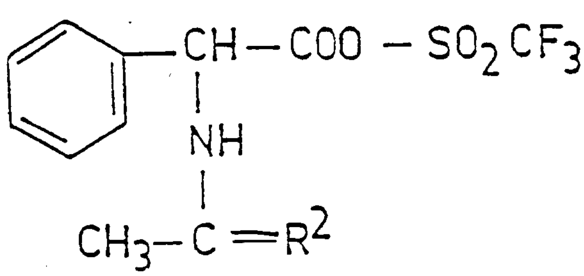

(VI)

worin

$R^2$ eine =CH-COOR-Gruppe - in der R ein $C_{1-2}$-Alkyl ist - oder eine Gruppe der Formel XI:

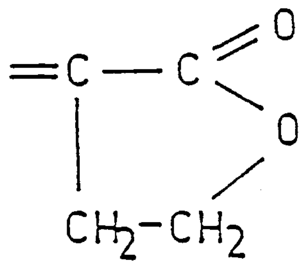

(XI)

bedeutet.

## Revendications

1. Procédé pour la préparation de composés de formule générale (I)

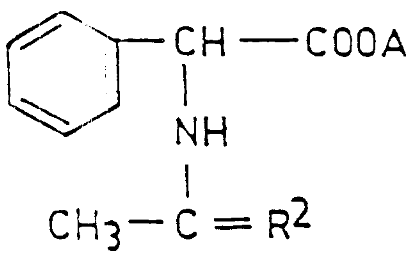

(I)

dans laquelle

A représente un atome d'hydrogène, Me ou un groupe $SO_2CF_3$, où Me est un ion potassium ou sodium

$R^2$ représente = CH - COOR ou un groupe de formule (XI)

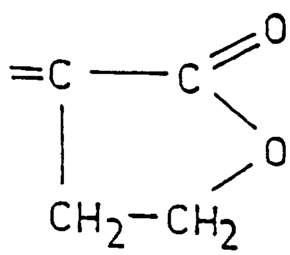

(XI)

R étant un groupe alkyle en $C_{1-2}$,

à partir d'un sel d'acide aminé de formule générale (II)

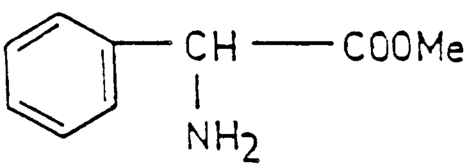

(II)

Me étant tel que donné ci-dessus -

qui comprend la réaction de la phénylglycine de formule (VII)

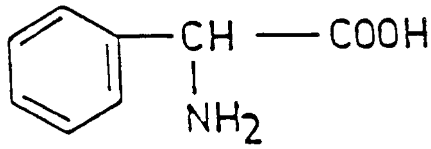

(VII)

en présence d'alcanol avec 1,05-1,3 moles d'hydroxyde alcalin pour former un sel et la réaction du mélange avec un acide ayant une valeur de pH supérieure à 4,3 et l'application de 1,06-1,4 moles d'équivalent d'acide calculé sur la phénylglycine, la dissolution de l'hydroxyde alcalin en excès de manière sélective, tandis que le sel de l'acide aminé de formule générale (II) - Me est tel que mentionné ci-dessus - précipite dans la solution, ou la réalisation de la réaction avec une quantité équivalente de sels alcalins d'acides possédant une valeur de pH supérieure à 4,3, et la condensation du sel de l'acide aminé de formule générale (II) obtenu de cette manière ou par un procédé connu en lui-même - Me est tel que mentionné ci-dessus-
en présence d'alcanol avec un composé de formule générale (VIII)

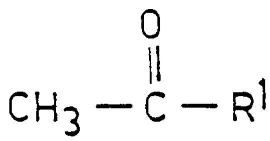

(VIII)

- $R^1$ représente - $CH_2$- COOR ou un groupe de formule (IX)

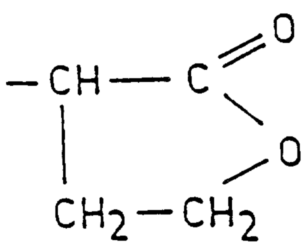

(IX)

- R a la même signification que mentionné ci-dessus -
  au point d'ébullition du mélange de réaction, tandis que l'eau formée dans la réaction - occasionnellement par l'addition d'un solvant- est éliminée en continu du mélange de réaction par une distillation azéotropique binaire ou ternaire, et l'isolement du sel condensé de formule générale (IV)

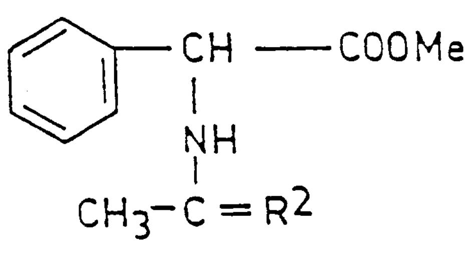

(IV)

- $R^2$ et Me sont tels que mentionnés ci-dessus - sous une forme cristalline à partir du mélange de réaction en refroidissant le mélange et le séchage et/ou la réaction du sel de formule générale (IV) obtenu de cette manière ou par un autre procédé - $R^2$ et Me sont tels que mentionnés ci-dessus - avec un dérivé réactif de l'acide trifluorométhanesulfonique de formule (V),

$F_3C$-$SO_2OH$ (V)

de préférence en présence d'un solvant polaire aprotique.

2. Procédé selon la revendication 1, caractérisé en ce que l'on sépare l'hydroxyde alcalin en excès par réaction du mélange de réaction avec l'acide acétique ou propionique.

3. Procédé selon la revendication 1 ou 2, caractérisé en ce que l'on effectue la condensation en présence d'alcool méthylique et lorsqu'on termine la réaction, on élimine l'alcool méthylique par distillation en donnant au mélange de réaction un solvant qui avec l'eau forme un mélange binaire de point d'ébullition plus et en ce qu'on élimine l'eau par distillation azéotropique.

4. Procédé selon la revendication 3, caractérisé en ce que l'on ajoute des esters inférieurs ou des hydrocarbures aromatiques, avantageusement le carbonate de diéthyle, les acétates d'alkyle, le benzène, le toluène, le xylène ou l'acétonitrile afin d'éliminer l'eau sous la forme d'un mélange binaire.

5. Procédé selon la revendication 1 ou 2, caractérisé en ce que l'on effectue la condensation en présence d'alcool éthylique et en ce que l'on élimine l'eau formée du mélange de réaction par une distillation azéotropique binaire.

6. Procédé selon la revendication 1 ou 2, caractérisé en ce que l'on effectue la condensation en présence d'alcool éthylique et qu'à la fin de la réaction on ajoute un solvant tel qu'il forme avec l'eau et l'alcool éthylique un mélange ayant un point d'ébullition minimal et en ce qu'on élimine l'eau par distillation azéotropique.

7. Procédé selon la revendication caractérisé en ce qu'en plus de l'alcool éthylique on utilise comme solvant l'acétate d'éthyle le benzène ou l'acétate de butyle.

8. Procédé pour la préparation de composés de formule générale (VI)

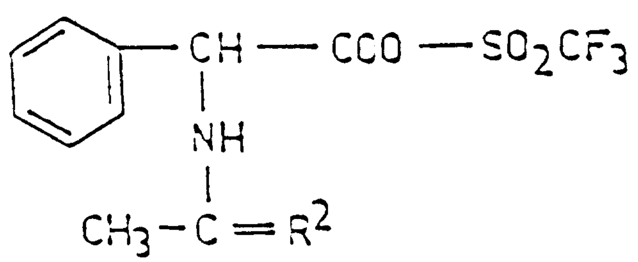

(VI),

dans laquelle $R^2$ représente = CH - COOR ou un groupe de formule (XI)

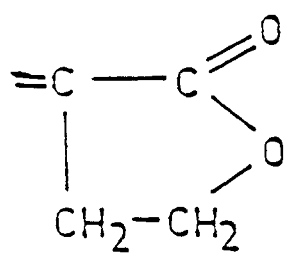

(XI),

où R désigne un groupe alkyle en $C_{1-2}$,
caractérisé en ce que l'on fait réagir un sel condensé de formule générale (IV)

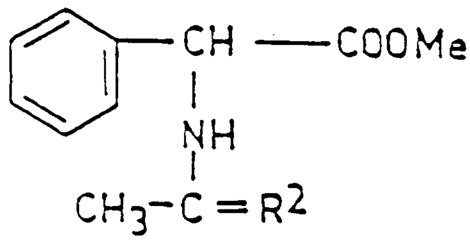

(IV),

dans laquelle $R^2$ possède la signification mentionnée ci-dessus et Me représente Na ou K, avec un dérivé réactif de l'acide trifluorométhanesulfonique, de préférence son chlorure.

**9.** Procédé selon la revendication 8, caractérisé en ce que l'on fait réagir le sel condensé à une température de 0-5° C avec le chlorure de l'acide trifluorométhanesulfonique.

**10.** Nouveaux composés de formule générale (VI)

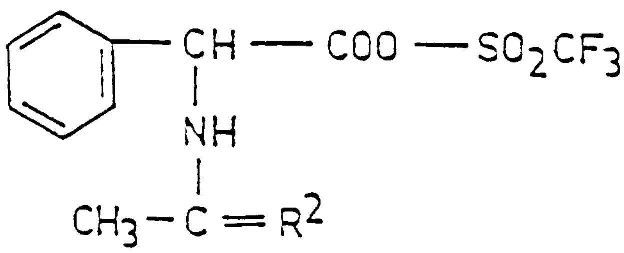

(VI),

dans laquelle $R^2$ représente = CH - COOR ou un groupe de formule (XI)

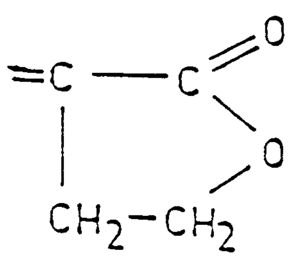

(XI),

et R représente un groupe alkyle en $C_{1-2}$.